# DEMANDE DE BREVET EUROPEEN

(11) **EP 4 837 586 A1**
(43) Date de publication de la demande: **11.11.2026**
(21) Numéro de dépôt: 26177243.8
(22) Date de dépôt: 07.05.2026
(51) Int. Cl.: A61K 36/9066, A61K 8/60, A61K 8/68, A61K 8/73, A61K 8/99, A61K 8/9728, A61K 8/9789, A61K 8/9794, A61K 31/702, A61K 31/715, A61K 45/06, A61P 17/02, A61Q 17/00, A61Q 19/00, A61Q 19/02, A61Q 19/08

(54) **COMPOSITION DERMATOLOGIQUE**

(30) Priorité: 07.05.2025 FR 2504871
(71) Demandeur: LABORATOIRES SVR, 91220 Le Plessis-Pâté (FR)
(72) Inventeur: TAMELGHAGHET, Myriam, 91760 ITTEVILLE (FR); COLLARD PEIGNE, Natacha, 78450 VILLEPREUX (FR)
(74) Mandataire: Cabinet Didier Martin

(57) **Abrégé**

L'invention concerne une composition dermatologique à usage topique caractérisée en ce qu'elle comprend au moins un extrait de Curcuma longa riche en exosomes végétaux naturels et des prébiotiques incluant au moins un oligosaccharide d'alpha-glucane et un polysaccharide riche en rhamnose.

## Description

La présente invention se rapporte au domaine technique général des produits dermatologiques, utilisables à des fins cosmétiques et/ou thérapeutiques, notamment pour améliorer la cicatrisation.

La présente invention concerne plus précisément une composition dermatologique à usage topique.

La peau peut subir différentes agressions (brûlures, blessures, actes dermatologiques, interventions chirurgicales...) aboutissant à une plaie. Un processus de cicatrisation se met alors en place, afin que la peau retrouve sa fonction de barrière vis-à-vis de l'environnement. Ce processus se déroule généralement en trois étapes interconnectées, savoir une première étape vasculaire et inflammatoire, une deuxième étape proliférative et enfin une troisième étape de maturation. Au cours de ces différentes étapes, un grand nombre de cellules et produits actifs (enzymes, protéines, cytokines...) sont mis en jeu. Plus précisément, la première étape précitée est constituée d'une phase vasculaire et d'une phase inflammatoire. Ainsi, lorsqu'une plaie affecte le derme, la coagulation est activée (phase vasculaire) pour stopper les éventuels saignements le plus vite possible et un caillot de fibrine (matrice provisoire) est formé. Ensuite, une réaction inflammatoire se déclenche pour nettoyer la plaie et lutter contre l'infection. Cette phase inflammatoire permet de préparer l'étape suivante, en recrutant les différentes cellules épidermiques. Lors de l'étape proliférative qui suit, la plaie se contracte, permettant le rapprochement des berges de la plaie, de sorte que cette dernière se referme petit à petit. L'épiderme et le derme se reconstruisent grâce à la production, par le derme adjacent, de fibres de collagène permettant la synthèse d'une nouvelle matrice tissulaire. Dès que la matrice en question est formée, la troisième et dernière étape de cicatrisation se met en place (étape de maturation). La plaie est alors fermée et le tissu de granulation (matrice tissulaire) se transforme en épithélium cicatriciel. L'épiderme est reconstruit entièrement pour acquérir sa composition classique. La peau se réorganise, les cellules inutiles meurent et les cellules bénéfiques vont se développer (élastine, collagène, réseau vasculaire).

Ces différentes étapes peuvent se dérouler plus ou moins rapidement, et conduire à un résultat final plus ou moins esthétique, en fonction notamment de différents facteurs tels que la localisation de la plaie, l'état général de la personne concernée et son mode de vie (facteurs nutritionnels, métaboliques, endocriniens, prise de médicaments...). En outre, avec l'âge, le processus de réparation des lésions cutanées peut s'avérer moins efficace et plus lent.

En définitive, compte tenu de ce qui précède, le résultat final d'un processus naturel de cicatrisation peut s'avérer défavorable sur le plan esthétique.

Les objets assignés à l'invention visent par conséquent à remédier aux différents inconvénients exposés précédemment, et à proposer une nouvelle composition dermatologique qui permet une réparation particulièrement rapide et esthétique des lésions cutanées.

Un autre objet de l'invention vise à proposer une nouvelle composition dermatologique qui favorise l'hydratation et la régénération de la peau dans le cadre d'un processus de cicatrisation.

Un autre objet de l'invention vise à proposer une nouvelle composition dermatologique qui agit favorablement sur l'inflammation ainsi que la régénération au niveau du derme et de l'épiderme, en maintenant et redensifiant la matrice extracellulaire et en régénérant et restructurant l'épiderme.

Un autre objet de l'invention vise à proposer une nouvelle composition dermatologique qui favorise la restauration de l'équilibre de la flore cutanée dans le cadre d'un processus de cicatrisation.

Un autre objet de l'invention vise à proposer une nouvelle composition dermatologique qui permet de limiter, voire même d'empêcher, d'éventuelles bactéries pathogènes d'adhérer à la surface de la peau dans le cadre d'un processus de cicatrisation.

Un autre objet de l'invention vise à proposer une nouvelle composition dermatologique qui favorise l'angiogenèse, la formation de tissus de granulation et la régénération des tissus, tout en maintenant un environnement humide propice à la cicatrisation et en stimulant les facteurs de croissance et la migration cellulaire essentiels à la réparation cutanée.

Un autre objet de l'invention vise à proposer une nouvelle composition dermatologique permettant d'améliorer l'intégrité de la barrière cutanée et d'augmenter l'hydratation de la peau au cours du processus de cicatrisation.

Un autre objet de l'invention vise à proposer une nouvelle composition dermatologique qui permet de prévenir et/ou de réduire les marques visibles d'hyper-pigmentation pouvant survenir lors du processus de cicatrisation.

Les objets assignés à l'invention sont atteints à l'aide d'une composition dermatologique à usage topique caractérisée en ce qu'elle comprend au moins :
- un extrait de *Curcuma longa* contenant des exosomes végétaux naturels, ledit extrait de *Curcuma longa* incluant un lysat de cellules souches totipotentes de callus de *Curcuma longa* ; et
- une première substance agissant sur le microbiome cutané incluant au moins un oligosaccharide d'alpha-glucane et une deuxième substance agissant sur le microbiome cutané incluant au moins un polysaccharide contenant du rhamnose.

D'autres particularités et avantages de l'invention apparaîtront et ressortiront plus en détail à la lecture de la description faite ci-après, donnée uniquement à titre illustratif et non limitatif.

L'invention concerne une composition dermatologique à usage topique, c'est-à-dire qui est conçue pour être appliquée directement sur la peau, à des fins cosmétiques et/ou thérapeutique. La composition dermatologique selon l'invention est avantageusement destinée à être utilisée pour la prévention et/ou la cicatrisation et/ou la réparation de lésions cutanées. Plus précisément, la composition dermatologique selon l'invention est destinée à être appliquée de préférence au niveau d'une lésion de la peau (plaie traumatique, chirurgicale ou chronique, brûlure UV ou chimique...) pour favoriser une cicatrisation rapide et esthétique. La composition dermatologique selon l'invention est en outre avantageusement destinée à améliorer l'hydratation de la peau et/ou à avoir un effet anti-microbien et/ou un effet anti-inflammatoire, pour favoriser une cicatrisation optimale.

À cette fin, la composition dermatologique selon l'invention comprend au moins un extrait de *Curcuma longa* contenant des exosomes végétaux naturels, et de préférence riche en exosomes végétaux naturels. De préférence, lesdits exosomes végétaux naturels sont présents dans ledit extrait de *Curcuma longa* selon une teneur qui est au moins égale à 10⁸ exosomes par millilitre dudit extrait. Le curcuma a démontré un potentiel significatif dans la cicatrisation de la peau et la réparation des plaies. Les principaux composés actifs du curcuma possèdent en effet des propriétés anti-inflammatoires, anti-oxydantes et anti-microbiennes qui contribuent à accélérer la cicatrisation des plaies. Ledit extrait de *Curcuma longa* favorise plus précisément une ré-épithélialisation plus rapide et une régulation de l'inflammation au cours du processus de cicatrisation. Ledit extrait de *Curcuma longa* est un dérivé de cultures de cellules totipotentes de *Curcuma longa.*

Selon l'invention, ledit extrait de *Curcuma longa* inclut un lysat de cellules souches totipotentes de callus de *Curcuma longa.* Avantageusement, ledit extrait de *Curcuma longa* est formé par un lysat de cellules souches totipotentes de callus de *Curcuma longa.* En d'autres termes, ledit extrait de *Curcuma longa* est un dérivé issu de la culture cellulaire de *Curcuma longa.* Il est par ailleurs parfaitement envisageable que ledit extrait inclue, en complément dudit lysat de cellules, les cellules souches totipotentes intégrales. L'utilisation de cultures de cellules végétales permet la production de composés d'intérêts car les cellules totipotentes dédifférenciées de la plante d'intérêt (en l'espèce *Curcuma longa*) sont soumises à des conditions de stress particulières afin de faire produire aux cellules un ou plusieurs composés d'intérêts. Il en résulte notamment un métabolome riche en facteurs cellulaires de réponse au stress (osmolites, diarylheptanoïdes...), en polysaccharides, acides gras libres, protéines de bas poids moléculaire. Cette culture cellulaire contient également en l'espèce des exosomes, et de préférence une forte concentration en exosomes (par exemple au moins égale à 10⁸ exosomes par millilitres de l'extrait, de préférence de l'ordre de 10⁹ exosomes par millilitres). Les exosomes se présentent sous la forme de petites vésicules extracellulaires pouvant contenir de l'ADN, de l'ARN, des curcuminoïdes, des flavonoïdes, des terpènes, des sucres et acides organiques. Ces exosomes permettent d'améliorer l'hydratation et la régénération de la peau, favorisant ainsi la cicatrisation de façon particulièrement efficace.

Avantageusement, ledit extrait de *Curcuma longa* inclut de la glycérine dans laquelle ledit lysat cellulaire (et éventuellement lesdites cellules souches totipotentes) est dispersé. La glycérine joue ainsi le rôle d'excipient, et permet d'améliorer la stabilité de l'extrait, qui se présente avantageusement sous la forme d'une suspension. La glycérine permet en outre d'améliorer l'hydratation de la peau.

En définitive, à son pourcentage d'objectivation, ledit extrait de *Curcuma longa* contenant des exosomes végétaux naturels (de préférence riche en exosomes végétaux naturels) mis en œuvre dans la composition dermatologique selon l'invention agit sur l'inflammation, en réduisant certains cytokines et marqueurs pro-inflammatoires avec et sans stress physiologique, agit également sur la régénération au niveau du derme et de l'épiderme, en maintenant et en redensifiant la matrice extracellulaire, ainsi qu'en régénérant et restructurant l'épiderme et agit sur l'hydratation de la peau.

Avantageusement, le pourcentage massique dudit extrait de *Curcuma longa* dans ladite composition dermatologique est compris entre 0,5 % et 5 %, de préférence entre 1 % et 2 %, ce qui permet d'obtenir un effet optimal au niveau du derme (saturation du derme) et de l'épiderme (régénération et effet anti-inflammatoire). De préférence, le pourcentage massique dudit lysat de cellules souches totipotentes de callus de *Curcuma longa* dans ladite composition dermatologique est compris entre 0,1 et 1%, de préférence sensiblement égal à 0,5%.

Par exemple, ledit extrait de *Curcuma longa* contenant des exosome végétaux naturels (de préférence riche en exosome végétaux naturels) est formé par le produit Turmeria Zen^{™} C-02 commercialisé par la société Vytrus Biotech S.A., société de droit espagnol sise C/Sant Gaietà 121, 2^{ème} étage, 08221 Terrassa (Barcelone), Espagne, identifiée par le code CINC (Chinese Ingredient Notification Code) 128796-04146-7926 (nom INCI : Extrait de racine de *Curcuma longa* (curcuma), glycérine, acide citrique).

La composition dermatologique à usage topique selon l'invention comprend en outre des substances agissant sur le microbiome cutané (par exemple des prébiotiques), et plus précisément des sucres agissant sur le microbiome cutané (par exemple des sucres prébiotiques). Ladite composition dermatologique inclut ainsi :
- une première substance agissant sur le microbiome cutané (formant par exemple un premier prébiotique) incluant au moins un oligosaccharide d'alpha-glucane et
- une deuxième substance agissant sur le microbiome cutané (formant par exemple un deuxième prébiotique) incluant un polysaccharide contenant du rhamnose, et de préférence riche en rhamnose.

Lesdites substances (par exemple prébiotiques) agissant sur le microbiome cutané jouent sur la préservation de la flore bactérienne, qui s'avère importante pour une bonne cicatrisation. Ainsi, lors des phases de réparation et de remodelage, au cours de la cicatrisation, la recolonisation bactérienne permet de réguler l'inflammation, en synergie avec ledit extrait de *Curcuma longa* contenant des exosomes végétaux naturels (et de préférence riche en exosomes végétaux naturels), et d'optimiser le temps et la qualité de la cicatrisation. À cette fin, l'oligosaccharide d'alpha-glucane aide à restaurer l'équilibre de la flore cutanée en stimulant sélectivement la flore saprophyte (bénéfique) au détriment de la flore opportuniste (pathogène) et en stimulant la libération de peptides antimicrobiens. Ledit polysaccharide contenant du rhamnose (et de préférence riche en rhamnose) forme quant à lui un film protecteur qui empêche le phénomène d'adhésion des bactéries pathogènes à la surface de la peau.

La combinaison de l'extrait de *Curcuma longa* contenant des exosomes végétaux naturels (et de préférence riche en exosomes végétaux naturels) et desdites substances (par exemples prébiotiques) agissant sur le microbiome cutané précitées (oligosaccharide d'alpha-glucane et polysaccharide contenant du rhamnose, et de préférence riche en rhamnose) permet un effet synergique conduisant à une réparation plus rapide, plus efficace et de meilleure qualité des lésions de la peau, en agissant simultanément sur trois axes majeurs de la cicatrisation :
- réduction rapide et durable de l'inflammation ;
- protection et équilibre optimal du microbiome cutané ;
- stimulation puissante et ciblée de la régénération cellulaire,
ainsi que sur l'hydratation de la peau.

Plus précisément, l'association de l'oligosaccharide d'alpha-glucane et du polysaccharide contenant du rhamnose (et de préférence riche en rhamnose) permet une restauration synergique du microbiome cutané, avec une action de restauration rapide de l'équilibre du microbiome cutané par l'oligosaccharide d'alpha-glucane, qui stimule sélectivement les bactéries bénéfiques, permettant une cicatrisation optimale, tandis que le polysaccharide contenant du rhamnose (et de préférence riche en rhamnose) bloque spécifiquement l'adhésion des bactéries pathogènes, limitant ainsi les infections et la perturbation du microbiome naturel. L'association desdites première et deuxième substances agissant sur le microbiome cutané (formant par exemple des premier et un deuxième prébiotiques) conduit ainsi à un environnement favorable à une cicatrisation saine, rapide et efficace.

En outre, la combinaison de l'extrait de *Curcuma longa* contenant des exosomes végétaux naturels (et de préférence riche en exosomes végétaux naturels) et du polysaccharide contenant du rhamnose (et de préférence riche en rhamnose) permet un contrôle synergique de l'inflammation, avec l'extrait végétal de *Curcuma longa* contenant des exosomes (et de préférence riche en exosomes) permettant de réduire l'inflammation initiale, tandis que le polysaccharide contenant du rhamnose (et de préférence riche en rhamnose) prévient toute réactivation inflammatoire liée à d'éventuelles infections secondaires, par inhibition de l'adhésion bactérienne. Ceci permet là encore de créer un environnement optimal pour une cicatrisation rapide et stable. L'extrait de *Curcuma longa* contenant des exosomes végétaux naturels et le polysaccharide contenant du rhamnose coopèrent aussi pour hydrater la peau de façon optimale.

Enfin, l'association de l'extrait végétal de *Curcuma longa* contenant des exosomes (et de préférence riche en exosomes) et de l'oligosaccharide d'alpha-glucane permet une amélioration synergique de la régénération cellulaire. En effet, en rétablissant rapidement un microbiome sain, l'oligosaccharide d'alpha glucane limite le stress microbien des cellules cutanées, optimisant de ce fait les conditions nécessaires à la prolifération et la différenciation cellulaire dans les tissus lésés, ce qui permet d'optimiser l'action des exosomes végétaux qui, en favorisant la signalisation cellulaire nécessaire à la régénération des kératinocytes et fibroblastes, stimulent la réparation épidermique et dermique. L'extrait de *Curcuma longa* contenant des exosomes végétaux naturels et l'oligosaccharide d'alpha-glucane coopèrent aussi pour hydrater la peau de façon optimale.

La combinaison de l'extrait de *Curcuma longa* contenant des exosomes (et de préférence riche en exosomes), et desdits sucres agissant sur le microbiome cutané (formant par exemple des sucres prébiotiques) permet ainsi une réparation plus rapide, plus efficace et de meilleure qualité des lésions de la peau, en agissant simultanément sur trois axes majeurs de la cicatrisation (inflammation, microbiome cutané et régénération cellulaire), ainsi que sur l'hydratation de la peau.

Avantageusement, le pourcentage massique de ladite première substance (par exemple prébiotique) agissant sur le microbiome cutané (à base d'oligosaccharide d'alpha-glucane) dans ladite composition dermatologique est compris entre 0,25 % et 3 %, de façon plus préférentielle entre 0,5 % et 1 %. Le recours à une telle proportion massique permet d'optimiser les effets techniques évoqués précédemment, et de favoriser en particulier la synergie avec le dérivé du *Curcuma longa* contenant des exosomes. De préférence, ledit oligosaccharide d'alpha-glucane présente une structure en chaine de résidus de glucose comportant des liaisons α-(1→6) et/ou des liaisons α-(1→2). Grâce à la présence desdites liaisons α-(1→6) et α-(1→2), la composition dermatologique selon l'invention permet une métabolisation sélective plus rapide et plus efficace du glucose par les micro-organismes présents sur la peau. Ladite première substance agissant sur le microbiome cutané est ainsi de préférence un prébiotique.

Avantageusement, la première substance agissant sur le microbiome cutané (formant par exemple un premier probiotique) incluant ledit oligosaccharide d'alpha-glucane est formé par le produit Bioecolia ^{®} commercialisé par la société Solabia, sise Liberty Tower - 17, place des reflets 92400 Courbevoie France. Le nom INCI de l'ingrédient Bioecolia ^{®} est alpha-glucan oligosaccharide.

Avantageusement, le pourcentage massique du rhamnose dans le polysaccharide contenant du rhamnose (et de préférence riche en rhamnose) est d'au moins 40 %, de préférence au moins 50 %, de préférence 60 %. Grâce à cette teneur élevée en rhamnose, ledit polysaccharide présente un pouvoir anti-inflammatoire élevé.

Avantageusement, ledit polysaccharide contenant du rhamnose (et de préférence riche en rhamnose), inclut en outre du glucose et/ou de l'acide glucuronique, et de préférence inclut à la fois du glucose et de l'acide glucuronique. Dans ce mode de réalisation préférentiel, ledit polysaccharide contenant du rhamnose (et de préférence riche en rhamnose) et incluant du glucose ainsi que de l'acide glucuronique forme un film protecteur à la surface de la peau qui limite l'adhésion des bactéries susceptibles d'entraîner un déséquilibre du microbiote. Cela permet d'empêcher tout contact entre ces bactéries et les cellules des récepteurs cutanés, conduisant à une inhibition de l'activation de la réponse immuno-inflammatoire. Ainsi ladite deuxième substance agissant sur le microbiome cutané forme avantageusement un deuxième prébiotique. Ledit polysaccharide concourt aussi à l'hydratation de la peau.

Par exemple, ledit polysaccharide contenant du rhamnose (et de préférence riche en rhamnose), contient 20 % en masse de glucose, et 20 % en masse d'acide glucuronique, ainsi que 60 % en masse de rhamnose.

Avantageusement, ladite deuxième substance agissant sur le microbiome cutané (formant par exemple un deuxième prébiotique) inclut, outre ledit polysaccharide contenant du rhamnose (et de préférence riche en rhamnose), de l'eau et du propanediol. Le propanediol joue le rôle d'excipient et permet d'améliorer la stabilité de ladite deuxième substance. Le propanediol permet en outre d'améliorer l'hydratation de la peau, notamment en synergie avec la glycérine contenue dans ledit extrait de *Curcuma longa,* lorsque celle-ci est présente ainsi qu'avec les exosomes végétaux naturels dudit extrait de *Curcuma longa.* Dans ce cas, le pourcentage massique dudit propanediol dans ladite deuxième substance agissant sur le microbiome cutané (formant par exemple un deuxième prébiotique) s'élève à 50 %, tandis que le pourcentage massique d'eau dans ladite deuxième substance agissant sur le microbiome cutané (formant par exemple un deuxième prébiotique) s'élève à 49 %, le polysaccharide représentant quant à lui un pourcentage massique de 1 % dans ladite deuxième substance agissant sur le microbiome cutané (formant par exemple un deuxième prébiotique), dont 0,6 % correspondant au rhamnose, 0,2 % correspondant au glucose et 0,2 % correspondant à l'acide glucuronique.

De préférence, en particulier dans le cas de figure précité où la deuxième substance agissant sur le microbiome cutané (formant par exemple un deuxième prébiotique) inclut 1 % de polysaccharide contenant du rhamnose (et de préférence riche en rhamnose), 49 % d'eau et 50 % de propanediol, le pourcentage massique de ladite deuxième substance agissant sur le microbiome cutané (formant par exemple un deuxième prébiotique) dans ladite composition est préférentiellement compris entre 0,1 % et 4 %, de préférence entre 0,1 % et 1 %, ce qui permet d'optimiser les différents effets exposés dans ce qui précède, en particulier les effets synergiques avec la première substance agissant sur le microbiome cutané (formant par exemple un premier prébiotique) et l'extrait de *Curcuma longa* contenant des exosomes, et de préférence riche en exosomes. De préférence, en particulier dans le cas de figure précité où la deuxième substance agissant sur le microbiome cutané contient 20 % en masse de glucose, et 20 % en masse d'acide glucuronique, ainsi que 60 % en masse de rhamnose, la fraction massique de rhamnose dans ladite composition dermatologique est comprise entre 6 et 240 ppm, de préférence entre 6 et 60 ppm ; la fraction massique de glucose dans ladite composition dermatologique est comprise entre 2 et 80 ppm, de préférence entre 2 et 20 ppm et la fraction massique d'acide glucuronique dans ladite composition dermatologique est comprise entre 2 et 80 ppm, de préférence entre 2 et 20 ppm.

Afin d'améliorer encore l'efficacité de la réparation cutanée, à tous les niveaux (épiderme, derme et hypoderme), la combinaison d'actifs exposés dans ce qui précède peut être complétée avec des actifs complémentaires. Ainsi, dans un mode de réalisation particulièrement avantageux, ladite composition dermatologique comprend également un hyaluronane, c'est-à-dire de l'acide hyaluronique, ou l'un de ses sels, ou l'un de ses dérivés. De préférence, ledit hyaluronane est formé d'acide hyaluronique hydrolysé. Ledit hyaluronane présente de préférence un poids moléculaire compris entre 100 et 300 kDa. De préférence, ledit hyaluronane est formé de chaînes de disaccharides, lesquelles sont composées d'acide D-glucuronique et de N-acétyl glucosamine, ledit hyaluronane étant de préférence formé de 300 à 600 répétitions d'unités disaccharidiques. Le recours à un acide hyaluronique hydrolysé permet de bénéficier de fragments polymériques plus courts que ceux de l'acide hyaluronique natif, avec un poids moléculaire plus bas (de préférence dans la plage susvisée), ce qui favorise une meilleure pénétration cutanée par rapport à la forme native plus volumineuse, tout en conservant une activité hydratante importante, ainsi qu'une capacité de production endogène d'acide hyaluronique dans le derme.

Avantageusement, le pourcentage massique dudit hyaluronane dans ladite composition dermatologique est compris entre 0,1 % et 0,5 %, et de préférence est égale à 0,2 %. Le recours à de l'acide hyaluronique en tant qu'actif supplémentaire de ladite composition dermatologique, en combinaison avec les autres actifs évoqués précédemment, notamment selon la proportion massique susvisée, permet d'obtenir différents avantages. En tant que composant majeur de la matrice extracellulaire, l'acide hyaluronique favorise l'angiogenèse, la formation du tissu de granulation et la régénération des tissus. Il maintient un environnement humide propice à la cicatrisation et stimule les facteurs de croissance et les migrations cellulaires essentiels à la réparation, qu'il s'agisse de réparer des plaies traumatiques, chirurgicale ou chronique, ou encore des brûlures, ou qu'il s'agisse de réduire les cicatrices. En particulier, lorsque la gamme de poids moléculaire susvisée est mise en œuvre, l'acide hyaluronique assure l'hydratation de l'épiderme, l'accélération de la réparation cutanée au niveau de l'épiderme (prolifération des kératinocytes) et favorise la réparation du derme (migration des fibroblastes) tout en améliorant le support structurel. Plus précisément, grâce à la gamme de poids moléculaire précitée, le dérivé d'acide hyaluronique susvisé est capable de pénétrer dans les couches profondes de la peau où il peut activer la réponse immunitaire et favoriser la libération de β-défensines 2 par les kératinocytes sans pour autant entraîner de réactions inflammatoires. L'acide hyaluronique permet ainsi un effet synergique avec l'extrait de *Curcuma longa* au niveau du derme, que l'acide hyaluronique peut atteindre grâce à son bas poids moléculaire.

Avantageusement, ladite composition dermatologique inclut en outre au moins un céramide. Ledit céramide est avantageusement un phytosphingolipide. La présence de cet actif supplémentaire dans la formulation permet d'améliorer l'intégrité de la barrière cutanée et d'augmenter l'hydratation de la peau, que cette dernière soit intacte ou compromise. Ledit au moins un céramide assure ainsi un effet synergique au niveau de l'épiderme avec l'extrait de *Curcuma longa* ainsi que de préférence l'acide hyaluronique. Avantageusement, ladite composition dermatologique inclut en outre un phytoglycolglycérolipide. De préférence, le pourcentage massique dudit phytosphingolipide dans ladite composition dermatologique est compris entre 0,03 et 0,05, de préférence entre 0,04 et 0,045%. De préférence, le pourcentage massique dudit phytoglycolglycérolipide dans ladite composition dermatologique est compris entre 0,03 et 0,05%, de préférence entre 0,035 et 0,040%.

De façon préférentielle, ladite composition dermatologique comprend un extrait végétal de céréales, de préférence un extrait végétal de blé, comprenant un mélange desdits phytosphingolipide et phytoglycolglycérolipide. Ledit extrait végétal de céréales se présente par exemple sous la forme d'une huile issue de blé contenant des céramides, de préférence riche en céramides, correspondant à un extrait de graines de *Triticum Aestivum* (Poaceae) obtenue par extraction au solvant (nom INCI : glycosphingolipids, glycolipids). Un tel extrait végétal de céréales favorise la fonction barrière au niveau de l'épiderme et contribue au renforcement de ce dernier, permettant ainsi un effet synergique avec en particulier l'extrait de *Curcuma longa* et les sucres agissant sur le microbiome cutané (formant par exemple des sucres prébiotiques). De préférence, le pourcentage massique dudit extrait végétal de céréales, et de préférence dudit mélange desdits phytosphingolipide et phytoglycolglycérolipide, dans ladite composition dermatologique est compris entre 0,05 % et 0,1 %, de préférence entre 0,08 % et 0,1 %, ce qui permet d'atteindre un effet synergique optimal.

Avantageusement, ladite composition dermatologique comprend en outre, en tant qu'actif additionnel, un postbiotique, qui est de préférence un postbiotique marin. De façon particulièrement avantageuse, ledit postbiotique comprend un extrait de ferment d'une souche de l'espèce *Eupenicillium crustaceum.* Ledit postbiotique forme avantageusement un actif agissant sur l'hyper-pigmentation post-inflammatoire, afin de prévenir et agir sur les marques visibles pouvant survenir avec le processus de cicatrisation. Afin d'obtenir un effet optimal à cet égard, le pourcentage massique dudit post-biotique dans ladite composition dermatologique est compris avantageusement entre 1 et 3 %, et est de préférence sensiblement égal à 2 %. Le postbiotique en question est formé par exemple par un mélange de butylène glycol, d'eau, d'extrait de levure hydrolysée et de glycolipides (nom INCI : butylène glycol, aqua, hydrolyzed yeast extract, glycolipids) qui permet une action éclaircissante et anti-tâches, en synergie avec les autres actifs évoqués dans ce qui précède. Dans ce cas, la fraction massique dudit ferment d'une souche de l'espèce *Eupenicillium crustaceum* (nom INCI : hydrolyzed yeast extract) dans ladite composition dermatologique est compris avantageusement entre 5 et 20 ppm, et est de préférence compris entre 10 et 12 ppm.

Avantageusement, ladite composition dermatologique comprend des excipients, par exemple au moins 90 % d'excipients, de façon encore plus préférentielle au moins 95 % d'excipients, lesdits excipients permettant de conférer à ladite composition dermatologique une forme galénique souhaitée, par exemple : émulsion, lotion aqueuse, produit anhydre (liste non limitative).

Un exemple de composition dermatologique selon l'invention, qui permet d'obtenir des résultats particulièrement remarquables en termes de rapidité de cicatrisation et sur le plan esthétique, est exposé dans le tableau 1 qui suit.

**Tableau 1**

| ***Nom commercial Matière*** | ***Noms INCI UE*** | ***Nom Fournisseur*** | ***% Massique*** |
|---|---|---|---|
| BIOECOLIA ^{®} | ALPHA-GLUCAN OLIGOSACCHARIDE | SOLABIA | 0,50 |
| TEFLOSE ^{®} | PROPANEDIOL AQUA RHAMNOSE GLUCOSE GLUCURONIC ACID | SOLABIA | 1,00 |
| PRIMALHYAL 300 ^{®} | HYDROLYZED HYALURONIC ACID | GIVAUDAN | 0,20 |
| TURMERIA ZEN^{™} C-02 | CURCUMA LONGA ROOT EXTRACT GLYCERIN CITRIC ACID | VYTRUS | 1,00 |
| CERAMOSIDES HP ^{®} | GLYCOSPHINGOLIPIDS GLYCOLIPIDS | SEPPIC | 0,08 |
| BRIGHLETTE MARINE ^{®} INGR. BI090 | BUTYLENE GLYCOL AQUA HYDROLYZED YEAST EXTRACT GLYCOLIPIDS | LIPOTEC | 2,00 |
| Excipients | | DIVERS | 95,22 |
| **TOTAL** | | | **100,00** |

La composition selon l'exemple exposé dans le tableau 1 ci-dessus a fait l'objet de différents tests sur des explants cutanés lésés (blessures mécaniques, brûlures par UV, brûlures chimiques) qui ont permis de mettre en évidence une action particulièrement efficace en matière de réparation cutanée.

### Test n°1 : Lésions mécaniques

### Test n°1 : Préparation des explants

Des explants de peau humaine blessée rectangulaires, de dimensions 10×15 mm, ont été préparés à partir d'une abdoplastie provenant d'une femme caucasienne âgée de 39 ans avec un phototype II selon la classification de types de peaux de Fitzpatrick. Les explants ont été maintenus en survie dans un milieu de culture approprié à 37°C, en atmosphère humide à 5 % de CO₂.

### Test n°1 : Répartition des explants

Les explants ont été distribués en lots selon le tableau 2 ci-après. La composition P correspond à la composition dermatologique du tableau 1 ci-avant.

**Tableau 2**

| **Lot** | **Désignation** | **Nombre d'explants** | **Echantillonnage** |
|---|---|---|---|
| B0 | Témoin lésion | 3 | Jour J0 |
| B | Témoin lésion | 6 | Jours J5 & J9 |
| BP | Lésion + Composition P | 6 | Jours J5 & J9 |

### Test n°1 : Lésions mécaniques

À J0, deux lésions par explant ont été pratiquées sur les explants rectangulaires 10×15 mm des lots B, en utilisant un poinçon de biopsie de 3,5 mm de diamètre. Les lésions ont été réalisées juste avant l'application de la composition P sur les lésions.

### Test n°1 : Application de la composition P

La composition P a été appliquée de façon topique sur la base de 3 µL par explant pour les lots BP (environ 2 mg/cm²) et étalée à l'aide d'une petite spatule à J0 (après la lésion), J2, J5 et J7.

### Test n°1 : Échantillonnage

À J5 et J9, des explants de chaque lot concerné ont été collectés et coupés chacun en deux parties. Une moitié a été fixée dans une solution tampon de formol et l'autre moitié a été congelée à -80°C.

### Test n°1 : Traitement histologique

Après fixation pendant 24 heures dans le formol tamponné, les échantillons ont été déshydratés et imprégnés de paraffine en utilisant un automate de déshydratation Leica Pearl. Les échantillons ont été enrobés à l'aide d'une station d'enrobage Leica EG 1160. Des sections de 5 µm d'épaisseur ont été réalisées en utilisant un microtome de type Minot Leica RM 2125, et les sections ont été montées sur des lames de verre histologique Superfrost ^{®}. Les échantillons congelés ont été découpés en sections de 7 µm d'épaisseur en utilisant un cryostat Leica CM 3050. Les sections ont été ensuite montées sur des lames de verre silanisé Superfrost ^{®} plus.

Des observations microscopiques ont été effectuées en utilisant un microscope Leica DMLB, Olympus BX43 ou BX63. Des clichés numériques ont été réalisés avec une caméra numérique Olympus DP72 ou DP74 avec le logiciel CellSens.

### Test n°1 : Morphologie générale

Une évaluation de l'aspect des bourgeons de croissance (zone lésée des lots blessés B), également appelé néo-épiderme, a été effectuée par observation microscopique. Certains paramètres ont été évalués en fonction des échelles et scores suivants, afin de décrire la structure des bourgeons de croissance, comme détaillé ci-après :
- Le nombre de couches de cellules, qui caractérise la prolifération des bourgeons de croissance, est évalué selon le nombre moyen de couches de cellules de bourgeon de croissance.
- L'adhésion au derme est évaluée sur une échelle de 0 à 5. Plus le score est élevé sur cette échelle, plus l'adhésion est élevée.

### Test n°1 : Résultats et discussions

Les scores des bourgeons de croissance formés pour les différents lots à J5 et J9 sont exposés dans le tableau 3 ci-après, étant entendu qu'aucun bourgeon de croissance n'a été observé à J0.

**Tableau 3**

| | **Score bourgeons de croissance** | |
|---|---|---|
| **Lots** | **Nombre de couches de cellules (prolifération)** | **Adhésion** |
| **BJ5** | 3 | 2 |
| **BPJ5** | 5 | 4 |
| **BJ9** | 4 | 2 |
| **BPJ9** | 5 | 2 |

Il ressort du tableau 3 ci-avant que la composition P du tableau 1 stimule significativement la prolifération des bourgeons de croissance après 5 jours (+ 66 % par rapport au témoin), ainsi qu'à 9 jours dans une moindre mesure (+ 25 % par rapport au témoin). On constate également une stimulation forte de l'adhésion des bourgeons de croissance après 5 jours (+ 100 % par rapport au témoin).

### Test n°2 : Brûlures par UV

### Test n°2 : Préparation des explants

Des explants de peau humaine circulaires, d'un diamètre moyen de 11 mm (+/- 1 mm) ont été préparés à partir d'une abdoplastie provenant d'une femme caucasienne âgée de 54 ans avec un phototype Il selon la classification de types de peaux de Fitzpatrick. Les explants ont été maintenus en survie dans un milieu de culture approprié à 37°C, en atmosphère humide à 5 % de CO₂.

### Test n°2 : Répartition des explants

Les explants ont été distribués en lots selon le tableau 4 ci-après. La composition P correspond à la composition dermatologique du tableau 1 ci-avant.

**Tableau 4**

| **Lot** | **Désignation** | **Nombre d'explants** | **Echantillonnage** |
|---|---|---|---|
| B0 | Témoin lésion UV-B 10 J/cm² | 3 | Jour J0 |
| B | Témoin lésion UV-B 10 J/cm² | 6 | Jours J5 & J9 |
| BP | Lésion UV-B 10 J/cm² + Composition P | 6 | Jours J5 & J9 |

### Test n°2 : Irradiations UV-B

À J0, tous les explants ont été mis dans 1 mL de solution saline équilibrée de Hanks (HBSS). Les explants des lots B ont été irradiés en utilisant un simulateur solaire Vilbert Lourmat RMX 3W, avec une dose de 10 J/cm² d'UV-B correspondant à 67 MED (Minimal Erythemal Dose - dose érythèmale minimale) pour créer une lésion de brûlure au centre des explants. Après les irradiations, tous les explants ont été replacés dans 2 mL du médium de culture.

### Test n°2 : Application de la composition P

La composition P a été appliquée de façon topique sur la base de 2 µL par explant et étalée à l'aide d'une petite spatule à J0 (après l'irradiation UV-B), J2, J5 et J7.

### Test n°2 : Échantillonnage

À J5 et J9, des explants de chaque lot concerné ont été collectés et coupés chacun en deux parties. Une moitié a été fixée dans une solution tampon de formol et l'autre moitié a été congelée à -80°C.

### Test n°2 : Traitement histologique

Après fixation pendant 24 heures dans le formol tamponné, les échantillons ont été déshydratés et imprégnés de paraffine en utilisant un automate de déshydratation Leica Pearl. Les échantillons ont été enrobés à l'aide d'une station d'enrobage Leica EG 1160. Des sections de 5 µm d'épaisseur ont été réalisées en utilisant un microtome de type Minot Leica RM 2125, et les sections ont été montées sur des lames de verre histologique Superfrost ^{®}. Les échantillons congelés ont été découpés en sections de 7 µm d'épaisseur en utilisant un cryostat Leica CM 3050. Les sections ont été ensuite montées sur des lames de verre silanisé Superfrost ^{®} plus.

Des observations microscopiques ont été effectuées en utilisant un microscope Leica DMLB, Olympus BX43 ou BX63. Des clichés numériques ont été réalisés avec une caméra numérique Olympus DP72 ou DP74 avec le logiciel CellSens.

### Test n°2 : Longueur des bourgeons de croissance

La longueur des bourgeons de croissance a été mesurée en utilisant le logiciel CellSens, sur la base de tous les lots B à l'exception de B0.

### Test n°2 : Résultats et discussions

Les longueurs moyennes des bourgeons de croissance formés pour les différents lots à J5 et J9 sont exposés dans le tableau 5 ci-après, étant entendu qu'aucun bourgeon de croissance n'a été observé à J0.

**Tableau 5**

| | **Longueurs des bourgeons de croissance (en µm)** | | | | | | | |
|---|---|---|---|---|---|---|---|---|
| **Lots** | **Mesure n*°*1** | **Mesure n°2** | **Mesure n°3** | **Mesure n°4** | **Mesure n°5** | **Mesure n°6** | **Longueur moyenne** | **Ecarttype** |
| **BJ5** | 140,4 | 150,1 | 74,3 | 90,9 | 84,9 | 122,1 | 110,4 | 31,5 |
| **BPJ5** | 170,5 | 200,8 | 172,5 | 123,0 | 113,7 | 105,4 | 147,7 µm | 38,8 |
| **BJ9** | 238,9 | 213,3 | 250,2 | 234,2 | 119,1 | 152,2 | 201,3 µm | 53,3 |
| **BPJ9** | 177,79 | 269,55 | 123,23 | 192,95 | 173,79 | 100,86 | 173,0 µm | 59,0 |

Ces données ont fait l'objet d'un test t de Student, qui donne la probabilité p pour que 2 lots soient significativement différents. Les résultats de cette analyse statistique sont retranscrits dans le tableau 6 ci-après.

**Tableau 6**

| ***p*** | **BJ5** | **BPJ5** | **BJ9** | **BPJ9** |
|---|---|---|---|---|
| **BJ5** | - | **0,099#** | **0,007**** | **0,053#** |
| **BPJ5** | **0,099#** | - | **0,077#** | 0,4030 |
| **BJ9** | **0,007**** | **0,077#** | - | 0,046 |
| **BPJ9** | **0,053#** | 0,4030 | 0,046 | - |

Si p<0,1 (#), la probabilité que 2 lots soient significativement différents est de 90%. Si p<0,05 (*), la probabilité que 2 lots soient significativement différents est de 95%. Si *p*<0,01 (**), la probabilité que 2 lots soient significativement différents est de 99%.

Il ressort des données et analyses ci-dessus que la composition P stimule de façon significative la longueur des bourgeons de croissance après 5 jours (+ 34 % par rapport au témoin). A J9, une baisse de 14% est observée mais elle n'est pas significative lorsque la peau est lésée par des UV.

### Test n°3 : Brûlures chimiques (acide sulfurique concentré)

### Test n°3 : Préparation des explants

Des explants de peau humaine rectangulaires, de dimensions 10×15 mm, ont été préparés à partir d'une abdoplastie provenant d'une femme caucasienne âgée de 39 ans avec un phototype II selon la classification de types de peaux de Fitzpatrick. Les explants ont été maintenus en survie dans un milieu de culture approprié à 37°C, en atmosphère humide à 5 % de CO₂.

### Test n°3 : Répartition des explants

Les explants ont été distribués en lots selon le tableau 7 ci-après. La composition P correspond à la composition dermatologique du tableau 1 ci-avant.

**Tableau 7**

| **Lot** | **Désignation** | **Nombre d'explants** | **Echantillonnage** |
|---|---|---|---|
| B0 | Témoin brûlure acide sulfurique 95% | 3 | Jour J0 |
| B | Témoin brûlure acide sulfurique 95% | 6 | Jours J5 & J10 |
| BP | Brûlure acide sulfurique 95% + Composition P | 6 | Jours J5 & J10 |

### Test n°3 : Exposition aiguë à l'acide sulfurique

À J0, les explants ont été traités de façon topique avec un papier buvard (7x5 mm) imbibé d'une solution d'acide sulfurique à 95% (VWR, réf : 20700.298) pour créer une lésion de brûlure au centre des explants. Le papier buvard a été retiré après 30s de contact avec la peau. Après essuyage, la composition P du tableau 1 ci-avant a été immédiatement appliquée et les explants ont été replacés dans 2 mL du médium de culture.

### Test n°3 : Application de la composition P

La composition P a été appliquée de façon topique sur la base de 3 µL par explant et étalée à l'aide d'une petite spatule à J0 (après l'irradiation UV-B), J3, J5 et J7.

### Test n°3 : Échantillonnage

À J5 et J10, des explants de chaque lot concerné ont été collectés et coupés chacun en deux parties. Une moitié a été fixée dans une solution tampon de formol et l'autre moitié a été congelée à -80°C.

### Test n°3 : Traitement histologique

Après fixation pendant 24 heures dans le formol tamponné, les échantillons ont été déshydratés et imprégnés de paraffine en utilisant un automate de déshydratation Leica Pearl. Les échantillons ont été enrobés à l'aide d'une station d'enrobage Leica EG 1160. Des sections de 5 µm d'épaisseur ont été réalisées en utilisant un microtome de type Minot Leica RM 2125, et les sections ont été montées sur des lames de verre histologique Superfrost ^{®}. Les échantillons congelés ont été découpés en sections de 7 µm d'épaisseur en utilisant un cryostat Leica CM 3050. Les sections ont été ensuite montées sur des lames de verre silanisé Superfrost ^{®} plus.

Des observations microscopiques ont été effectuées en utilisant un microscope Leica DMLB, Olympus BX43 ou BX63. Des clichés numériques ont été réalisés avec une caméra numérique Olympus DP72 ou DP74 avec le logiciel CellSens.

### Test n°3 : Morphologie générale

En raison de la diffusion passive d'acide sulfurique à travers tout l'explant de peau durant la phase *ex vivo,* on n'observe ni zone périlésionnelle ni bourgeon de croissance. Afin d'évaluer l'effet de la composition P sur la lésion, la morphologie générale des zones lésées a été évaluée par observations microscopiques de sections de peau fixées dans le formol et enrobées de paraffine (FFPE) après coloration trichromique de Masson selon la variante Goldner.

### Test n°3 : Résultats

Les observations microscopiques effectuées ont mis en évidence, sur une peau brûlée par des agents chimiques, une diminution de la nécrose des tissus par coagulation à J5 et J10 (lots BPJ5 et BPJ10) par rapport aux témoins BJ5 et BJ10. En outre, à J5 la couche cornée du lot BPJ5 est mieux stratifiée par rapport au témoin BJ5, et à J10 on observe pour le lot BPJ10 une moindre altération de la matrice extracellulaire dermique par rapport au témoin BJ10.

## Revendications

1. Composition dermatologique à usage topique **caractérisée en ce qu'**elle comprend au moins :
- un extrait de *Curcuma longa* contenant des exosomes végétaux naturels, ledit extrait de *Curcuma longa* incluant un lysat de cellules souches totipotentes de callus de *Curcuma longa ;*
- une première substance agissant sur le microbiome cutané incluant au moins un oligosaccharide d'alpha-glucane et une deuxième substance agissant sur le microbiome cutané incluant au moins un polysaccharide contenant du rhamnose.

2. Composition dermatologique selon la revendication précédente **caractérisée en ce que** ledit extrait de *Curcuma longa* inclut de la glycérine dans laquelle ledit lysat est dispersé.

3. Composition dermatologique selon l'une quelconque des revendications précédentes **caractérisée en ce que** le pourcentage massique dudit lysat de cellules souches totipotentes de callus de *Curcuma longa* dans ladite composition dermatologique est compris entre 0,1% et 1%, de préférence sensiblement égal à 0,5%.

4. Composition dermatologique selon l'une quelconque des revendications précédentes **caractérisée en ce que** le pourcentage massique dudit oligosaccharide d'alpha-glucane dans ladite composition dermatologique est compris entre 0,25% et 3%, de préférence entre 0,5% et 1%.

5. Composition dermatologique selon l'une quelconque des revendications précédentes **caractérisée en ce que** ledit oligosaccharide d'alpha-glucane présente une structure en chaîne de résidus de glucose comportant des liaisons α-(1→6) et/ou des liaisons α-(1→2).

6. Composition dermatologique selon l'une quelconque des revendications précédentes **caractérisée en ce que** la fraction massique de rhamnose dans ladite composition dermatologique est comprise entre 6 et 240 ppm, de préférence entre 6 et 60 ppm.

7. Composition dermatologique selon l'une quelconque des revendications précédentes **caractérisée en ce que** ledit polysaccharide contenant du rhamnose inclut en outre du glucose et/ou de l'acide glucuronique.

8. Composition dermatologique selon l'une quelconque des revendications précédentes **caractérisée en ce que** ladite deuxième substance agissant sur le microbiome cutané inclut, outre ledit polysaccharide contenant du rhamnose, de l'eau et du propanediol.

9. Composition dermatologique selon l'une quelconque des revendications précédentes **caractérisée en ce qu'**elle comprend également un hyaluronane, avec un poids moléculaire compris de préférence entre 100 et 300 kDa, formé de préférence d'acide hyaluronique hydrolysé.

10. Composition dermatologique selon la revendication précédente **caractérisée en ce que** le pourcentage massique dudit hyaluronane dans ladite composition dermatologique est compris entre 0,1% et 0,5%, de préférence est sensiblement égal à 0,2%.

11. Composition dermatologique selon l'une quelconque des revendications précédentes **caractérisée en ce qu'**elle inclut au moins un céramide, ledit au moins un céramide étant un phytosphingolipide.

12. Composition dermatologique selon l'une quelconque des revendications précédentes **caractérisée en ce qu'**elle inclut un phytoglycolglycérolipide.

13. Composition dermatologique selon les revendications 11 et 12 **caractérisée en ce qu'**elle comprend un extrait végétal de céréales comprenant un mélange desdits phytosphingolipide et phytoglycolglycérolipide.

14. Composition dermatologique selon l'une quelconque des revendications précédentes **caractérisée en ce qu'**elle comprend en outre un postbiotique, de préférence un postbiotique marin, ledit postbiotique comprenant de préférence un extrait de ferment d'une souche de l'espèce *Eupenicillium crustaceum.*

15. Composition dermatologique selon l'une quelconque des revendications précédentes destinée à être utilisée pour la prévention et/ou la cicatrisation et/ou la réparation de lésions cutanées.
